# EUROPEAN PATENT APPLICATION

(11) **EP 3 075 408 A1**
(43) Date of publication of application: **05.10.2016**
(21) Application number: 14866138.2
(22) Date of filing: 27.11.2014
(51) Int. Cl.: A61M 25/02

(54) **SUPPORT FOR HOLDING THE EXTERNAL DEVICE OF A PERITONEAL DIALYSIS CATHETER, AND ADDITIONAL SYSTEM FOR ATTACHING THE SUPPORT**

(30) Priority: 28.11.2013 ES 201331372 U
(71) Applicant: Navarro Mallach, José, 46015 Valencia (ES)
(72) Inventor: Navarro Mallach, José, 46015 Valencia (ES)
(74) Representative: Dosterschill, Peter
(86) International application number: PCT/ES2014/070870
(87) International publication number: WO 2015/079089

(57) **Abstract**

The invention relates to a support (1) comprising a U-shaped enveloping body (1) having an elastic structure formed by a front limb (1 a), a rear limb (1 b) and a concave bottom (1c), where the U-shaped enveloping body (1) defines an inner space (9) where the external device (3) of the catheter fits tightly. The support (1) also has at least one extension (4) which projects from one of the limbs of the U-shaped enveloping body (1), where said limb and the extension (4) face each other, forming a clip device (5) for attaching the support to the patient's garment, the clip device (5) being formed outside the inner space (9) of the U-shaped enveloping body (1).

## Description

### OBJECT OF THE INVENTION

The present invention, as expressed in the title of this descriptive report, refers to a support to attach the external device of a peritoneal dialysis catheter used by people undergoing said treatment, wherein the external device of the catheter is used to connect the dialysis device to said device by means of the IV line previously inserted in the user's body at the height of the abdomen. Furthermore, the object of the invention is an additional attachment system of the external device support.

Based on this premise, the object of the invention is a peculiar support that allows for attaching the external device of the catheter safely and has means to attach the support itself to a user's garment, and even to a neck chain that the user would wear.

### BACKGROUND OF THE INVENTION

At present, a patient who has to live with a catheter to undergo the peritoneal dialysis treatment must have it hanging and projecting from the IV line in the waist area.

This way of carrying the external device of the catheter is very uncomfortable and unsafe for the patient, as during its movement, the external device of the catheter moves uncontrollably, and undesired tugging of the IV line due to unwanted hitching of said device to any garment or accessory that the user may carry, or even to any external element, may take place.

### DESCRIPTION OF THE INVENTION

With the aim of accomplishing safety, hygiene and comfort-related goals of the patient, and preventing some inconveniences concerning life quality, the invention proposes a support to attach the external device of a peritoneal dialysis catheter comprising:
- A U-shaped enclosing body bearing an elastic structure made up of a front branch, a rear branch and a curved-concave bottom, wherein the U-shaped enclosing body defines an inner space where the external device of the catheter is press-fitted.
- At least one extension that stretches out from one of the branches of the U-shaped enclosing body, wherein this branch and the extension are facing each other forming a clip device for attaching the support to a patient's garment, the clip device being formed outside the inner space of the U-shaped enclosing body.

In one embodiment, the extension stretches out from an end of one of the branches of the U-shaped enclosing body.

The inner sides of the two front and rear branches include several ridges intended for retaining the external device of the catheter.

The sides of the extension and branch of the U-shaped enclosing body facing each other include protrusions intended for reinforcing the attachment of the support to the garment.

The upper ends of the two front and rear branches of the U-shaped enclosing body are rounded.

The extension stretches out from an end of one of the branches of the U-shaped enclosing body.

On the other hand, when the user is not wearing clothes where to anchor the support of the external device of the catheter, an additional attachment system has been foreseen to be able to attach said support properly, according to several embodiments described below. This additional attachment system offers further confidence to the user and makes regular use of the external device of the catheter more comfortable.

In a first embodiment, a lower area of the extension comprises a through hole meant for hanging the support by means of a chain coupled to the user's neck.

In a second embodiment, the support is hung through a cord coupled to the user's neck by means of a magnetised element embedded in a through bore of the extension; wherein at least a section of this cord is a ferromagnetic material so that the magnetised element of the support is adhered thereto.

In a third embodiment the additional attachment system of the support comprises a dressing that is stuck to the user's body, said dressing including a ferromagnetic element so that a magnetised element included in the support is adhered thereto.

Said dressing comprises a laminar body, one of the sides of which has an adhesive material meant for sticking to the user's body, so that the ferromagnetic element is placed in mutual position with said side having the adhesive material. In a fourth embodiment, the support is hung from a clip tied to a cord coupled around the user's neck; wherein said clip includes a ferromagnetic element to which a magnetised element included in the support is adhered, the cord having two end sections where the clip is anchored through a blocking mechanism.

The clip has an inner cavity connected to the outside through two side windows through which the two end sections of the cord are inserted for their anchoring; said inner cavity being defined by a base and a movable body having an end tab that is snap-fitted in an L-shaped portion of the base when pressed on said movable body against the base of the clip; wherein the end sections of the cord inserted in the inner cavity of the clip are immobilised by means of an inner crossbar of the movable body which presses against said end sections resting on an inner side of the clip's base.

Inside the inner cavity of the clip, two side tracks are formed as guiding means of the end sections of the cord into said inner cavity; wherein said tracks stretch out from the side windows and converge into an inner cavity area facing the inner crossbar of the movable body.

Hereinafter, in order to give a better understanding of this description, the object of the invention has been detailed in a series of drawings that are an integral part of the report and are for illustration purposes and without limitation.

### BRIEF DESCRIPTION OF THE DRAWINGS

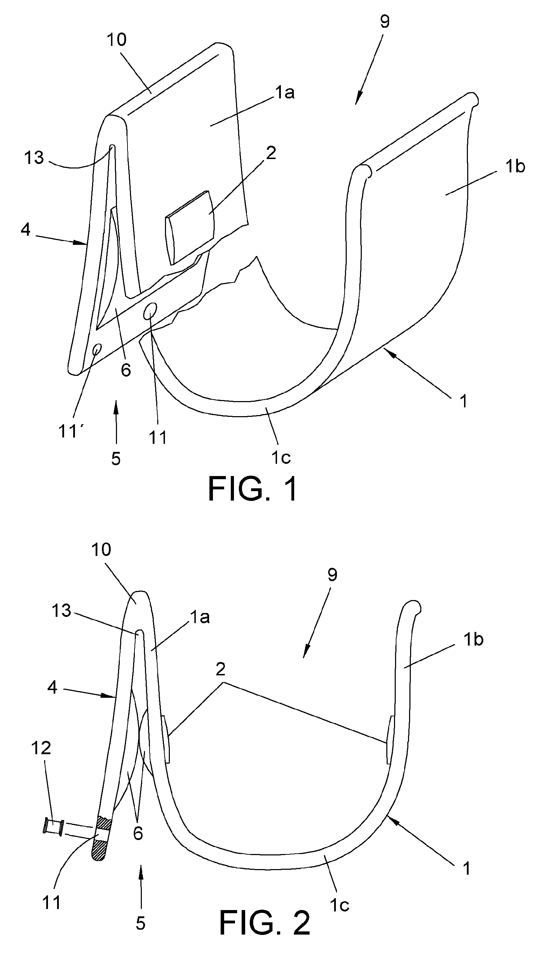

- Figure 1: shows a perspective view of the support to attach the external device of a peritoneal dialysis catheter, which is the object of the invention, an additional attachment system of the support also being an object of the invention.
- Figure 2: shows an elevation view of what has been represented in the previous figure.
- Figure 3: shows a plane view where the application of the support that is the object of the invention is shown.
- Figure 4: shows a perspective view of the additional attachment system of the support.
- Figure 5: shows a profile view of what has been represented in the previous figure.
- Figure 6: shows a perspective view of another additional attachment system of the support.
- Figure 7: shows a profile view of what has been represented in the previous figure.

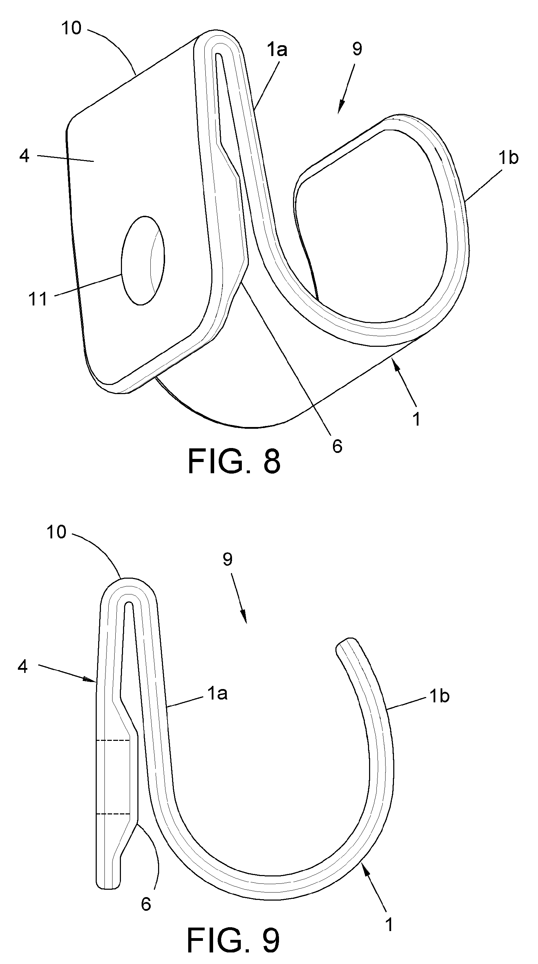

- Figure 8: shows a perspective view of the support that is the object of the invention.
- Figure 9: shows an elevation view of what has been represented in the previous figure.

### DESCRIPTION OF A SAMPLE EMBODIMENT OF THE INVENTION

Considering the numbering adopted for the figures, the support to attach the external device of a peritoneal dialysis catheter features the following nomenclature used in the description:
1.- U-shaped enclosing body.
1 a.- Front branch.
1 b.- Rear branch.
1 c.- Curved-concave bottom.
2.- Ridges.
3.- External device.
4.- Extension.
5.- Clip device.
6.- Protrusions.
7.- First IV line.
8.- Second IV line.
9.- Inner space.
10. Junction area.
11.- Through bore.
11'.- Through hole.
12.- Magnetised element.
13.- Bottom.
14.- Dressing.
15.- User's body.
16.- Ferromagnetic element.
17.- Laminar body.
18.- Pocket.
19.- Clip.
20.- Cord.
20a.- End section.
21.- Base.
21 a.- L-shaped portion.
22.- Movable body.
23.- Inner crossbar.
24.- Side tracks.

It comprises a U-shaped enclosing body (1) bearing an elastic structure made up of a front branch (1 a), a rear branch (1 b) and a curved-concave bottom (1 c), the inner sides of both branches (1 a), (1 b) including several ridges (2) to hold the external support (3) of the peritoneal dialysis catheter more firmly, as described hereinafter.

An end of the front branch (1a) of the U-shaped enclosing body (1) stretches downwards forming an extension (4), said extension and said front branch (1a) making up a clip device (5) for attaching the support assembly by clipping to a user's garment, such as underwear, trousers, skirt, bathing suit, etc. The junction area (10) of the front branch (1 a) and extension (4) is an elastic joint, so the clip device (5) would always tend to close.

In one embodiment, the sides of the extension (4) and the front branch (1a) facing each other include several protrusions (6) intended for reinforcing the attachment of the clip device (5).

In another embodiment, the support of the invention is attached to the garment resting thereof by gravity, so that a bottom (13) of the clip device abuts on an edge of the garment. Accordingly, if the patient who carries the support of the invention removes the garment without unhitching the support from the tip of the catheter, and the unhitching would not take place, the non-inclusion of said protrusions (6) would make it easier for the whole support together with the catheter tip to unhitch jointly from the garment preventing any tugging or damage to the patient.

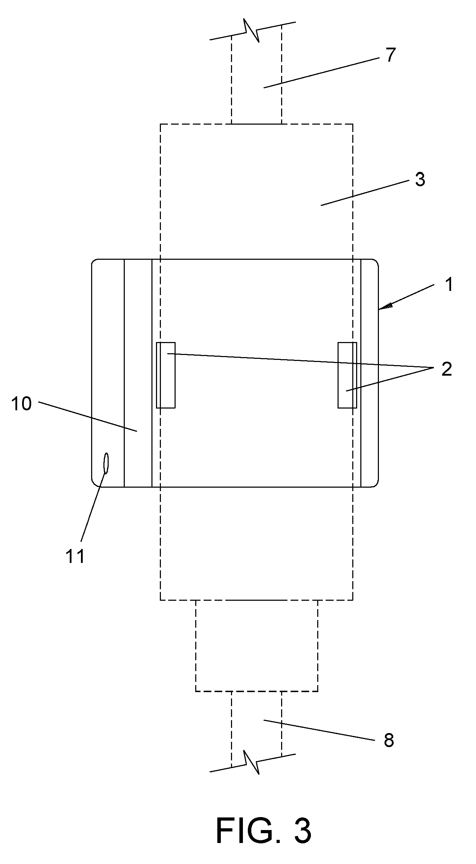

As it is more clearly shown in Figure 3, an end of the external device (3) of the catheter is connected with a first IV line (7) which extends from the user's body whereas the opposite end of the external device (3) of the catheter is connected with an end of a second IV line (8) which ends in a dialysis device not illustrated in the figures or rather a plastic plug when the external part of the catheter is not connected.

The external device (3) of the catheter is adjusted in an inner space (9) defined by the U-shaped enclosing body, so that the elasticity thereof in combination with the ridges (2) of its two branches (1a), (1 b) provide a firm, stable and safe attachment and immobilisation of the external device (3) of the catheter.

On the other hand, when the user is not wearing clothes where to anchor the support (1) of the external device (3) of the catheter, an additional attachment system has been foreseen to be able to attach said support (1), according to several embodiments described below. This additional attachment system provides greater confidence to the user and makes regular use of the external device (3) of the catheter more comfortable.

In a first embodiment, a lower area of the extension (4) includes a through hole (11') to be able to hang the support (1), for example, from a neck chain, so that said support (1) is not hanging and projecting while the patient is in the shower or not wearing any garment for its attachment.

In a second embodiment, the support (1) is hung from a cord around the patient's neck through a magnetised element (12) embedded in the through bore (11) of the extension (4). In this case, at least one section of the cord around the neck is made of a ferromagnetic material so that it joins the magnetised element (12) of the support (1).

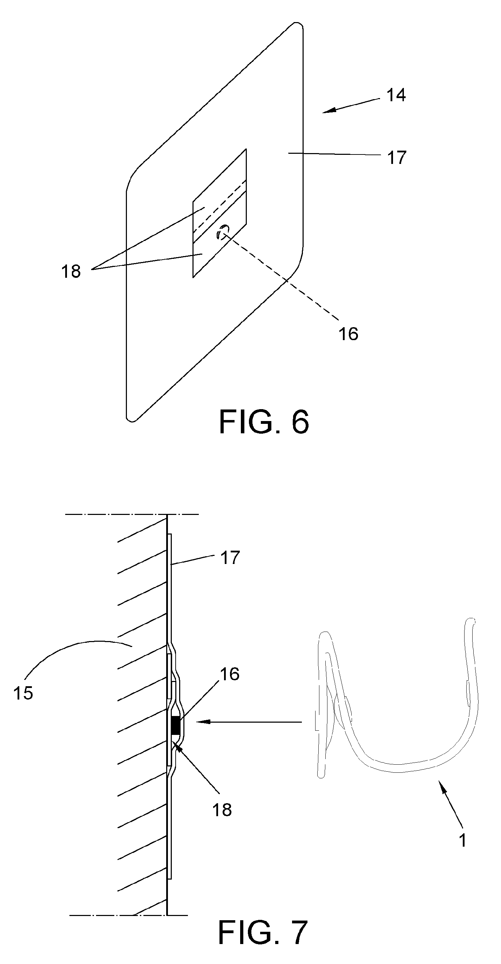

In a third embodiment, as shown in figures 6 and 7, the additional attachment system comprises a dressing (14) that is stuck to the user's body (15), said dressing (14) including a ferromagnetic element (16) to which the magnetised element (12) of the support (1) is joined, thus securing attachment. Said dressing (14) comprises a laminar body (17), one of the faces of which has an adhesive material for sticking to the user's body (15), and, in mutual position with said side is placed the ferromagnetic element (16), which, in one embodiment, is concealed inside a pocket (18) arranged in mutual position with said adhesive material side of the laminar body (17).

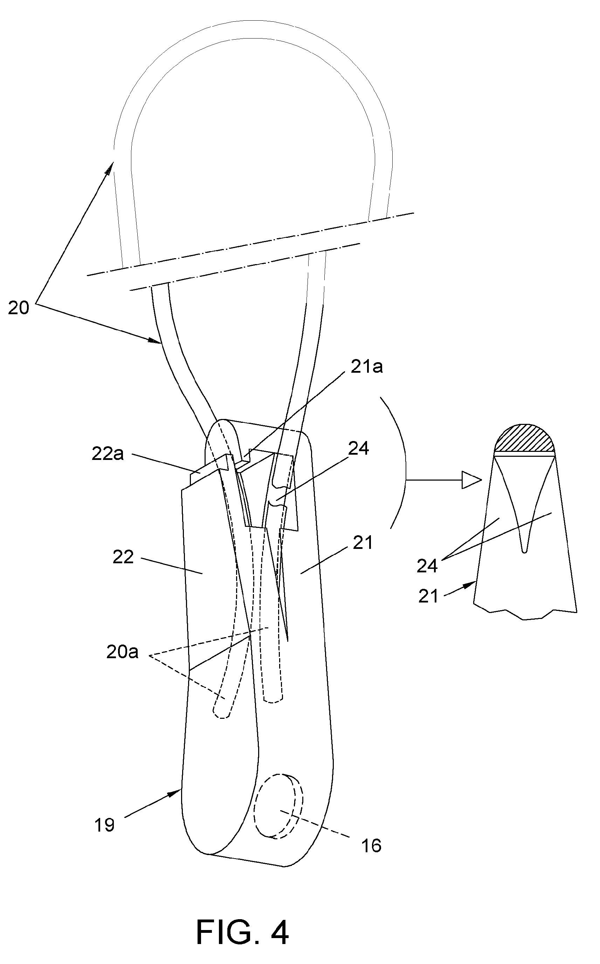

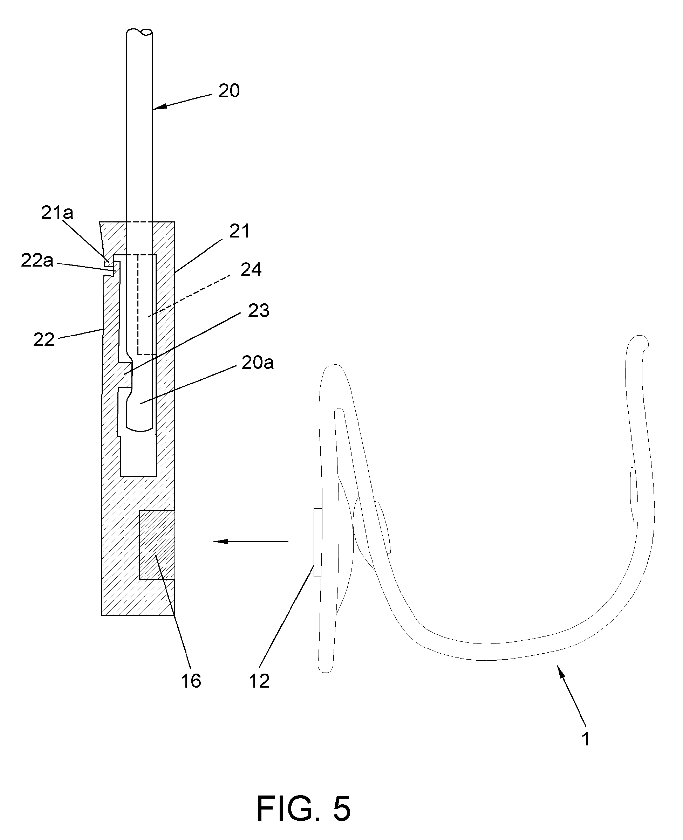

In a fourth embodiment, as shown in figures 4 and 5, the support (1) is hung from a clip (19) tied to a cord (20) coupled around the user's neck.

For such purpose, the clip (19) includes a ferromagnetic element (16) to which the magnetised element (12) of the support (1) is adhered or joined thus securing its attachment. In this case, the cord (20) has two end sections (20a) to which the clip (19) is anchored by means of a blocking mechanism, so that when an accidental tugging takes place, at least one end section (20a) of the cord (20) is released when subjected to a pulling force which would put the assembly of the external device (3) of the catheter at risk.

In reference to the above-said paragraph, the clip (19) includes an inner cavity that is connected to the outside through two side windows through which the two end sections (20a) of the cord (20) are inserted for their anchoring. Said inner cavity is defined by a base (21) and a movable body (22) having an end tab (22a) that is snap-fitted in an L-shaped portion (21 a) of the base (21) when pressed on said movable body (22) against the base (21) of the clip (14). In this case, the end sections (22a) of the cord (20) inserted in the inner cavity of the clip (14) are immobilised by means of an inner crossbar (23) rigidly mounted onto the movable body (22) which presses against the end sections (20a) of the cord (20) resting on the inner side of the base (21).

Inside the inner cavity of the clip (14), two side tracks (24) are formed to guide the end sections (20a) of the cord (20) into said inner cavity, so that said side tracks (24) stretch out from the side windows and converge into an inner cavity area facing the inner crossbar (23) of the movable body (22).

When the cord (20) is subjected to an excessive pulling force, as mentioned above, at least one of the end sections (20a) of the cord (20) is released and potential damage to the external device (3) as well as to the user is prevented.

As the U-shaped enclosing body (1) is open at the top (mouth) and at the ends, the external device (3) of the catheter exits or is easily removed from the support of the invention, in case the patient takes off his/her clothes and said clothes tended to drag the support, thus preventing tugging which may affect the entrance area of the first IV line (7) which stretches out from the user's or patient's body.

Ultimately, with the device of the invention, quality of life is improved as the inconveniences derived from carrying this catheter 24 hours a day are eliminated or minimised. Likewise, safety is enhanced as unwanted tugging is prevented, and hygiene is also improved as it is possible to carry the external device (3) of the catheter in a clean area. As it is located near the abdomen, the natural condition of use of the external device (3) of the catheter is to have it concealed by the underwear. With the support of the invention, placing options in other locations, such as onto the underwear, are feasible.

The upper ends of the two front (1 a) and rear (1 b) branches of the U-shaped enclosing body are rounded to facilitate fitting of the external device (3) of the catheter inside the inner space (9) of the support of the invention.

It is worth mentioning that in all the embodiments in which the magnetised elements (12) and the ferromagnetic elements (16) are used as attachment means, it is possible to exchange the location thereof, i.e. in the case of the dressing (14), it may include the magnetised element and the ferromagnetic element may be included in the U-shaped enclosing body (1). Another possibility is that the attachment means are two magnetised elements. Nevertheless, in patients with pacemakers it is not advisable to use magnetised elements included in the dressing (14), or in the cord hanging from the user's neck, or in the clip (19); all of which to prevent disturbances with said pacemaker.

## Claims

1. A support to attach the external device of a peritoneal dialysis catheter which, being intended for attaching the external device of a peritoneal dialysis catheter externally, is characterised as it comprises:
- a U-shaped enclosing body (1) bearing an elastic structure made up of a front branch (1 a), a rear branch (1 b) and a curved-concave bottom (1 c), wherein the U-shaped enclosing body (1) defines an inner space (9) where the external device (3) of the catheter is press-fitted;
- at least one extension (4) that stretches out from one of the branches of the U-shaped enclosing body (1), wherein this branch and the extension (4) are facing each other forming a clip device (5) to attach the support to a patient's garment, the clip device (5) being formed outside the inner space (9) of the U-shaped enclosing body (1).

2. A support to attach the external device of a peritoneal dialysis catheter, according to Claim 1, **characterised in that** the inner sides of the two front (1 a) and rear (1 b) branches of the U-shaped enclosing body (1) include several ridges (2) facing each other intended for retaining the external support (3) of the catheter.

3. A support to attach the external device of a peritoneal dialysis catheter, according to any of the previous claims, **characterised in that** at least one of the sides of the extension (4) and the branch of the U-shaped enclosing body (1) facing each other includes a protrusion (6) meant for reinforcing the attachment of the support to the garment.

4. A support to attach the external device of a peritoneal dialysis catheter, according to any of the previous claims,
**characterised in that** the upper ends of the two front (1a) and rear (1 b) branches of the U-shaped enclosing body (1) are rounded.

5. A support to attach the external device of a peritoneal dialysis catheter, according to any of the previous claims, **characterised in that** the extension (4) stretches out from one end of one of the branches of the U-shaped enclosing body (1).

6. An additional attachment system of the support of the external device, as described in any of the previous claims 1 to 5, **characterised in that** a lower area of the extension (4) includes a through hole (11') intended for hanging the support (1) by means of a chain coupled to the user's neck.

7. An additional attachment system of the support of the external device, as described in any of the previous claims 1 to 5, **characterised in that** the support (1) is hung from a cord coupled to the user's neck by means of a magnetised element (12) embedded in a through bore (11) of the extension (4); wherein at least one section of the cord is a ferromagnetic material so that it joins said magnetised element (12) of the support (1).

8. An additional attachment system of the support of the external device, as described in any of the previous claims 1 to 5, **characterised in that** it comprises a dressing (14) that is stuck to the user's body (15), said dressing (14) including a ferromagnetic element (16) so that a magnetised element (12) included in the support (1) is adhered thereto.

9. An additional attachment system of the support of the external device, according to claim 8, **characterised in that** the dressing (14) comprises a laminar body (17), one of the sides of which has an adhesive material meant for sticking to the user's body (15), the ferromagnetic element (16) being placed in mutual position with the same side having the adhesive material of said laminar body (17).

10. An additional attachment system of the support of the external device, as described in any of the previous claims 1 to 5, **characterised in that**:
- the support (1) is hung from a clip (19) tied to a cord (20) coupled around the user's neck;
- said clip (19) includes a ferromagnetic element (16) to which a magnetised element (12) included in the support (1) is adhered, wherein the cord (20) has two end sections (20a) where the clip (19) is anchored by means of a blocking mechanism.

11. An additional attachment system of the support of the external device, according to claim 10, **characterised in that**:
- the clip (19) includes an inner cavity that is connected to the outside through two side windows through which the two end sections (20a) of the cord (20) are inserted for their anchoring;
- said inner cavity is defined by a base (21) and a movable body (22) having an end tab (22a) that is snap-fitted in an L-shaped portion (21 a) of the base (21) when pressed on said movable body (22) against the base (21) of the clip (19); wherein the end sections (20a) of the cord (20) inserted in the inner cavity of the clip (14) are immobilised by means of an inner crossbar (23) of the movable body (22) which presses against said end sections (20a) resting on an inner side of the base (21) of the clip (14).

12. An additional attachment system of the support of the external device, according to claim 11, **characterised in that** inside the inner cavity of the clip (14), two side tracks (24) are formed to guide the end sections (20a) of the cord (20) into said inner cavity; wherein said tracks (24) stretch out from the side windows and converge into an inner cavity area facing the inner crossbar (23) of the movable body.
